# EUROPÄISCHE PATENTSCHRIFT

(11) **EP 2 338 563 B1**
(45) Veröffentlichungstag und Bekanntmachung des Hinweises auf die Patenterteilung: **20.11.2013**
(21) Anmeldenummer: 10192891.9
(22) Anmeldetag: 29.11.2010
(51) Int. Cl.: A61N 1/37

(54) **MRT-Signalisierung**
MRI signaling
Signalisation IRM

(30) Priorität: 22.12.2009 US 288859 P
(43) Veröffentlichungstag der Anmeldung: 29.06.2011
(73) Patentinhaber: BIOTRONIK CRM Patent AG, 6341 Baar (CH)
(72) Erfinder: Dörr, Thomas, 12437, Berlin (DE); Weiss, Ingo, 12435, Berlin (DE)
(74) Vertreter: Lindner-Vogt, Karin L.

(56) Entgegenhaltungen:
- WO-A1-2007/124273
- WO-A2-2006/124481
- US-A1- 2005 070 975
- US-A1- 2009 138 058

## Beschreibung

Die Erfindung bezieht sich auf eine Vorrichtung zum Umgang mit elektromagnetischen Feldern, speziell solchen Feldern, wie sie bei bildgebenden Kernspintomographie- (im folgenden MRT- oder MRI-) Geräten auftreten.

Obwohl MRI-Untersuchungen einen immer höheren Stellenwert in der diagnostischen Medizin erhalten, ist ein Teil der Patienten für MRI-Untersuchungen kontraindiziert. Eine solche Kontraindizierung kann durch ein mindestens teilweise implantiertes medizinisches Gerät (im Folgenden auch Implantat oder IMD) gegeben sein.

Um MRI-Untersuchungen dennoch zu ermöglichen, sind verschiedene Ansätze, die sich entweder auf die Durchführung der MRI-Untersuchung oder auf das implantierbare medizinische Gerät beziehen, bekannt.

So offenbart die US 2005/0070787, dass eine Kommunikation zwischen einem MRI-Gerät und einem Implantat etabliert wird, so dass das Implantat mit Informationen vom MRI-Gerät in der Lage ist, einen oder mehrere Komponenten des Implantats während der Abgabe von RF-Pulsen durch das MRI-Gerät zu deaktivieren. Dazu ist es wie beschrieben notwendig, dass eine Kommunikation zwischen Implantat und MRI-Gerät stattfindet, was ein gemeinsames Kommunikationsprotokoll und jeweils kompatible Kommunikationsfähigkeiten voraussetzt. Neben den damit notwendigen Kompatibilitätsvoraussetzungen und zusätzlich notwendigen Kommunikationseinheiten, speziell auf Seiten der MRI-Geräte, ist ein weiterer Nachteil die lange Übergangszeit, bis ein Großteil der MRI-Geräte eine sichere MRI-Untersuchung von Implantatsträgern zulässt.

Ein ähnlicher Ansatz wird in der EP 2 062 525 verfolgt. Dort wird ein RFID-System an zu einem Implantat gehörenden Elektrodenleitungen beschrieben, die einen Informationsfluss vom RFID-Kennzeichen zu einem MRI-Gerät zulässt. Bei diesem System sind also MRI-Geräte notwendig, die über eine Einheit zum Auslesen von RFID-Kennzeichnungen verfügen. Neben den damit notwendigen Kompatibilitätsvoraussetzungen und zusätzlich notwendigen RFID-Einheiten, speziell auf Seiten der MRI-Geräte, ist ein weitere Nachteil die lange Übergangszeit, bis ein Großteil der MRI-Geräte durch vorhandene RFID-Leseeinheiten eine sichere MRI-Untersuchung von Implantatsträgern zulässt.

Des Weiteren bedingen beide Systeme aus dem Stand der Technik, dass das jeweilige MRI-Gerät mit den aktuellen Informationen zu den jeweiligen Implantaten ausgestattet ist.

Des Weiteren beschreibt die US2009/0138058A1 die Möglichkeit der Kommunikation eines Implantates mit einem MRI-Gerät über eine Telemetrieschnittstelle. Um diese Kommunikation zu ermöglichen, müssen das MRI-Gerät und Implantat über eine kompatible Kommunikationseinheit verfügen.

Aufgabe der Erfindung ist es, die Nachteile des Stands der Technik zu beheben und ein implantierbares medizinisches Gerät (IMD) zur Verfügung zu stellen, dass eine sichere Untersuchung mit MRI-Methoden ermöglicht.

Die Aufgabe wird durch ein implantierbares medizinisches Gerät (IMD) mit den Merkmalen des Anspruchs 1 gelöst.

Die Erfindung ermöglicht, dass ein Implantat die für die Patientensicherheit erforderlichen Informationen an ein MR-Gerät sendet das sich daraufhin automatisch an die jeweiligen Implantatssicherheitsspezifikationen anpasst und/oder den Nutzer am Bildschirm auf das Implantat und/oder die notwendigen Einstellungen zur sicheren Verwendung aufmerksam macht. Die Informationen können auch über das Timing der Sequenzen hinaus auch Angaben zur max. erlaubten Leistung, max. Scannzeit und das wiederum in Abhängigkeit der Patientenlage im Scanner beinhalten. Die Leistungseinschränkung kann dabei sowohl für das RF- als auch das Gradientensystem übermittelt werden und ist im Allgemeinen abhängig von der statischen Magnetfeldstärke.

Die Übermittlung der Information, im einfachsten Fall auch nur die Signalisierung seiner Anwesenheit durch Erzeugung einer gewollten Bildstörung, kann dabei auf Initiative des Implantats erfolgen. Eine weitere erfindungsgemäße Lösung sieht vor, dass diese Information auf Initiative des MRI-Gerätes abgefragt wird, im Speziellen realisiert als passive RFID (Radio Frequency Identification).

Bevorzugt wird, dass der Informationsspeicher Daten und/oder Parameter nach Anspruch 2 enthält

Daten beziehen sich in diesem Zusammenhang auf Daten des IMD, während sich die Parameter auf technische oder physiologische Parameter der MRI-Untersuchung beziehen. Physiologische Parameter wären beispielsweise die Lage des Patienten, die erlaubten Untersuchungsbereiche, Lage des Implantats und/oder von Implantatsteilen, wie den Elektroden. Prinzipiell sind beide Informationen geeignet, eine sichere MRI-Untersuchung zu gewährleisten, wobei besonders die Übertragung von Parametern bevorzugt wird.

Ebenfalls bevorzugt ist, dass das dritte Signal mindestens einen der folgenden Werte und/oder Parameter umfasst, maximal zulässige RF Leistung, maximal zulässige Gradientenleistung, maximal zulässige Scandauer, gegebenenfalls in Abhängigkeit von einem oder mehreren der anderen Parameter, Patientenlage, maximal zulässige Untersuchungszeit und/oder Parameter zur Synchronisation von MRI-Scans und/oder MRI-Auslesephasen und IMD Aktivität bezogen auf Signaldetektion und/oder Therapieabgabe.

Ebenfalls besonders bevorzugt wird, dass das IMD die Larmorfrequenz aus einer Messung eines statischen Magnetfeldes und/oder der Frequenz der vorhergehenden elektromagnetischen Störung mittels der Einheit zur Detektion von elektromagnetischen Störfeldern bestimmt.

Auch wird besonders bevorzugt, dass das IMD die Trägerfrequenz und/oder Modulationstyp und/oder Modulationsfrequenz und/oder Modulationstiefe des von der Kommunikationseinheit initiierten Signals zur Bildstörung sich aus den Messungen der Einheit zur Detektion von elektromagnetischen Störfeldern berechnet.

Auch wird bevorzugt, dass das IMD keine interne Stromversorgung aufweist oder diese im Falle einer Erkennung einer elektromagnetischen Störung nicht zur Verfügung steht und/oder zur Entlastung einer vorhandenen Batterie auf die Nutzung der Batterie zur Versorgung der Kommunikation mit einem MRI-Gerät verzichtet wird, wobei das besagte dritte Signal mit der Kommunikationseinheit gesendet wird, indem die durch das MRI-Gerät induzierte Energie verwendet wird, um den Energiebedarf der Kommunikationseinheit zum Senden des besagten dritten Signals an das MRI-Gerät zu decken.

Ebenfalls bevorzugt wird, dass die Antenne oder Antennen aus langgestreckten Subsystemen, wie, aber nicht beschränkt auf, Elektroden von Herzschrittmachern, Defibrillatoren/Cardiovertern, Geräten zur kardialen Resychronisation oder Neurostimulatoren, und/oder einer bereits integrierten Antenne zur RF-Kommunikation bestehen.

Ebenso wird bevorzugt, dass das IMD mindestens eine weitere der folgenden Maßnahmen bei einer Erkennung von elektromagnetischen Störfeldern einleitet, das Wechseln in einen MRI-sicheren Zustand, ein verlängertes Verbleiben in einem MRI-sicheren oder gegenüber elektromagnetischen Störfeldern unempfindlichen Zustand, und die Abgabe einer Therapie und/oder Detektion von elektrischen Zuständen des Gewebes nur in Zeitfenstern, in denen keine elektromagnetischen Störfelder erkannt werden.

Des Weiteren wird bevorzugt, dass die Einheit zur Detektion von elektromagnetischen Störfeldern mindestens einen der folgenden Sensoren oder Indikatoren umfasst, GMR-Sensor, MagFET-Sensor, Hallsensor, elektrooptische Wandler als Indikator, die Überwachung von Batteriespannungen während Kondensatorladeprozessen als Indikator, die Detektion von RF-Feldern als Indikator, die Detektion von magnetischen Grandientenfeldern als Indikator, die Detektion von durch elektromagnetische Felder induzierten Strömen als Indikator, die Detektion von spezifischen Vibrationen oder als Sensoren ausgelegte Bauteile zur Detektion von durch Lorentzkräfte induzierten Vibrationen als Indikator. Unter Indikatoren sind dabei Verfahren und/oder Vorrichtungen zu verstehen, die Rückschlüsse auf das Vorhandensein von elektromagnetischen Störfeldern zulassen.

Besonders bevorzugt wird, dass zusätzlich zu mindestens einem der oben genannten Sensoren oder Indikatoren ein Lagesensor oder selbstkalibrierender Lagesensor vorhanden ist. Dieser oder diese Lagesensoren liefern mit der Bestimmung der Positur bzw. der Lage des Patienten einen zusätzlichen Indikator, ob sich der Patient in einem MRI-Gerät befindet oder andere elektromagnetische Störquellen vorliegen.

Neben den bisher genannten Vorrichtungen sind auch Verfahren zur Verwendung eines IMD im Wirkungsbereich eines MRI-Gerätes relevant, die ein IMD nach einer der vorangegangenen Ansprüche verwenden.

Einige Aspekte der Erfindung werden in den Figuren 1 -4 dargestellt.
- Fig. 1: schematische Darstellung des Ablaufs einer MRI-Untersuchung
- Fig. 2: schematische Darstellung eines erfindungsgemäßen Implantats mit ausgewählten Komponenten
- Fig. 3: schematische Darstellung eines erfindungsgemäßen Implantats mit ausgewählten Komponenten in Kommunikation mit einem MRI-Gerät
- Fig. 4: schematische Darstellung einer besonderen Ausgestaltung eines erfindungsgemäßen Implantats.

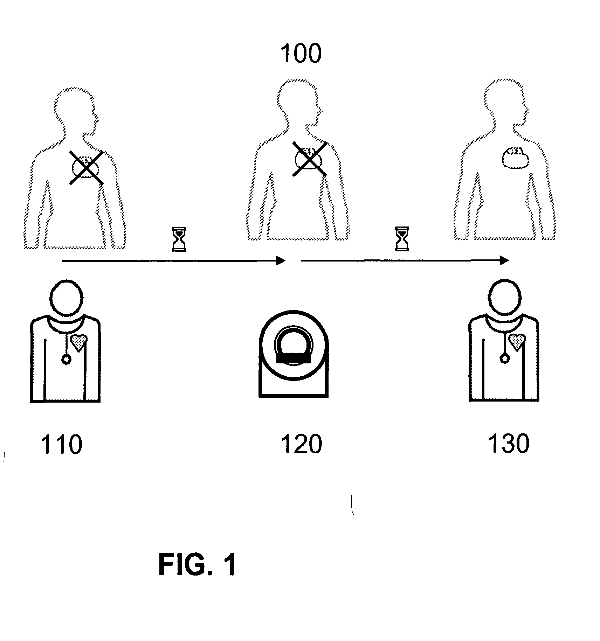

Figur 1 beschreibt den Stand der Technik, bei dem der ICD-Patient (100) vor der geplanten MRT-Untersuchung von einem Kardiologen nachgesorgt und der ICD ausgeschaltet (110) wird. Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen.

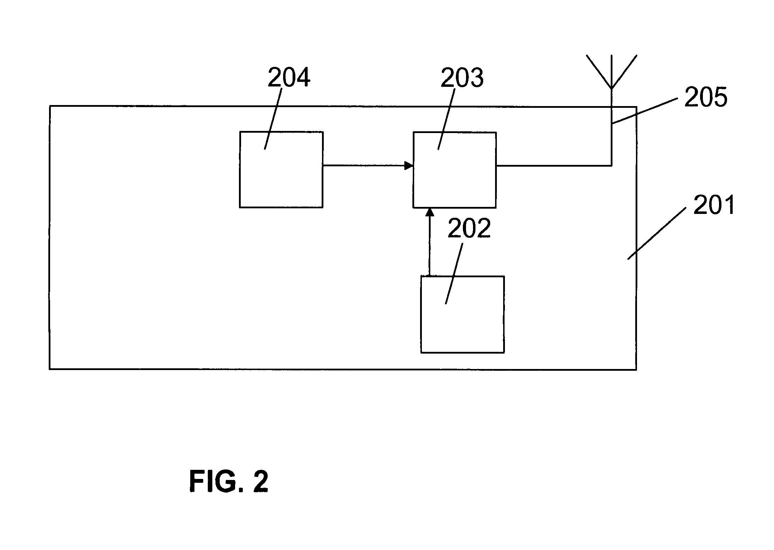

Figur 2 zeigt eine schematische Darstellung eines erfindungsgemäßen Implantates 201 mit einigen relevanten Komponenten. Erkennt die Störfelderkennungseinheit 204 eine elektromagnetische Störung, so wird ein Signal an die Kommunikationsvorrichtung 203 gegeben. In einer bevorzugten Ausführung wird das Signal nur beim Erreichen und/oder Überschreiten einer vorbestimmbaren Schwelle und/oder anderer die Störung charakterisierender Eigenschaften generiert. Nach Erhalt eines entsprechenden Signals über eine erkannte Störung von der Störfelderkennungseinheit 204 sendet die Kommunikationseinheit 203 über eine Antenne 205 ein Signal an das MRI-Gerät.

Alternativ kann die Kommunikationseinheit 203 in Verbindung mit der Antenne 205 auch eine bidirektionale Verbindung zum Datentransfer ermöglichen, speziell in diesen Fällen ist eine genaue Erkennung der Quelle der elektromagnetischen Störungen nicht zwingend notwendig, da sich in diesem Fall die Störquelle gegenüber dem Implantat identifizieren kann.

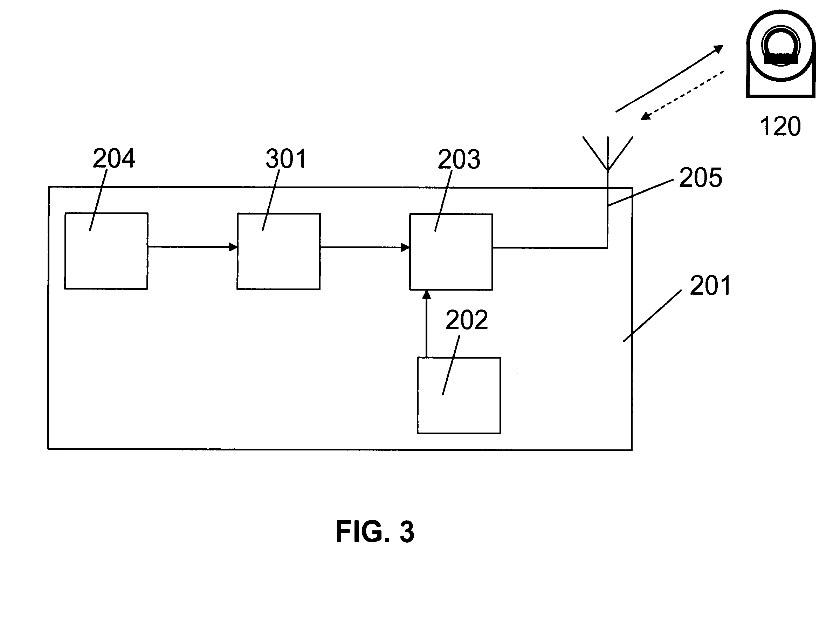

Die Figur 3 zeigt ein anderes Ausführungsbeispiel, in dem die Interaktion von Implantat und MRI-Gerät schematisch dargestellt wird.

Erkennt die Störfelderkennungseinheit 204 eine elektromagnetische Störung, so wird ein Signal über ein Verzögerungsglied 301 an die Kommunikationsvorrichtung 203 gesendet. Die Kommunikationseinheit sendet daraufhin Daten aus dem Speicher 202 über die Antenne 205. Durch das Verzögerungsglied 301 trifft das Signal des Implantats in die Auslesephase oder Detektionsphase des MRI-Gerätes und macht so durch Störung der Detektion und/oder Auslesung auf das Implantat aufmerksam. Alternativ kann das Signal des Implantats aber nicht nur eine Störung, z. B. Bildstörung an das MRI-Gerät übermitteln, sondern auch gezielt Informationen auf dem vom MRI-Gerät generierten Bild darstellen, z. B. dezidierte Anweisungen zum sicheren Vornehmen von MRI-Untersuchungen. Diese Anweisungen können sich unter anderem auf maximal zulässige Intensitäten, Einwirkzeiten oder die Koordinierung von Implantatstätigkeit und MRI-Untersuchung beziehen. Auch kann das System im Fall einer zulässigen Störung, das heißt, dass zum Beispiel die vorbestimmbaren Schwellwerte bei einer MRI-Untersuchung nicht erreicht oder überschritten werden, dafür genutzt werden, andere kritische Informationen, zum Beispiel über den Zustand des Implantatträgers, speziell bei Ereignissen, die eine Verschlechterung des Gesundheitszustandes und/oder einen lebensbedrohlichen Zustand herbeiführen, an das MRI-Gerät oder die Störquelle zu übermitteln.

Wie bereits darauf hingewiesen wurde, kann in einer Ausführungsform die vom Implantat ausgehende Störung entweder abschaltbar sein und/oder die Störung wird bei der MRI-Untersuchung berücksichtigt und herausgerechnet und/oder die Störung wird eingestellt, wenn die vorbestimmbaren Schwellwerte nicht überschritten und/oder erreicht und/oder Charakteristika nicht mehr erfüllt werden und die Störung somit als mit der Implantats-funktion kompatibel eingestuft wird.

Die Antenne 205 kann sowohl eine bereits vorhandene RF-Antenne und/oder eine eigens für den Zweck vorgesehene Antenne sein. Insbesondere können auch Elektrodenleitungen als Antennen verwendet werden.

In einer weiteren Ausführung erfolgt das Aussenden des Signalisierungssystems nur, wenn die MR Sensoren im Implantat einen Schwellwert erfüllen, insbesondere wenn der Scanner sich richtig angepasst hat, ist der Schwellwert nicht mehr erfüllt.

In einer weiteren Ausführung trägt das Implantat eine RFID, die genau zu diesem Zweck gedacht ist, dem MRI-Scanner die nötigen Informationen, insbesondere für Sicherheit, zu übermitteln.

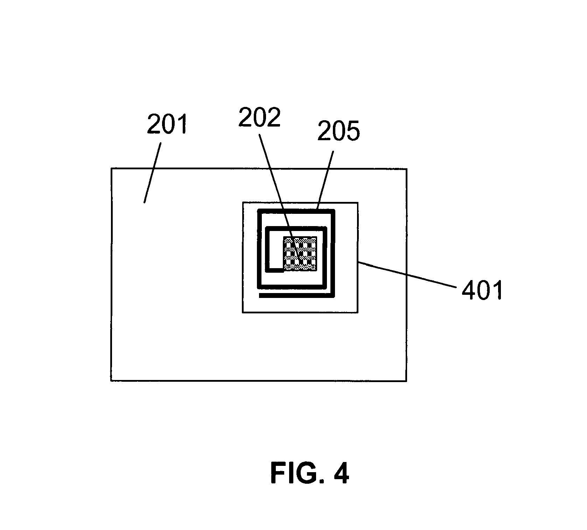

Figur 4 Zeigt eine spezielle erfindungsgemäße Lösung in Form eines Implantates 201 mit einer RFID-Kennzeichnung 401, die mit einem Speicher 202 und einer Antenne 205 verbunden ist. Ist das Implantat einer elektromagnetischen Störquelle, z. B. einem MRI-Gerät, ausgesetzt, die die voreinstellbaren Spezifikationen der RFID-Kennzeichnung erfüllt, so sendet die RFID-Kennzeichnung 401 vorbestimmbare Daten aus dem Speicher mit sicherheitsrelevanten Informationen 202 über die Antenne 205 aus. Diese Daten zeugen bei der Bilddarstellung eine Störung, wobei die Störung selbst geeignet ist, Informationen bei der Bilddarstellung wiederzugeben.

Besonders bevorzugt wird bei allen Ausführungsvarianten, dass die MRI-Erkennung möglichst früh vorgenommen wird und die Beeinflussung des MRI-Gerätes möglichst früh vorgenommen wird. Die Beeinflussung kann dabei bereits durch eine Bildstörung gegeben sein. Möglichst früh kann in dem Zusammenhang bedeuten, dass die Signalisierung oder Beeinflussung bereits bei einem Übersichtsscan oder der Initialisierung des MRI-Gerätes vorgenommen wird.

## Patentansprüche

1. Implantierbares medizinisches Gerät (IMD) mit einem Informationsspeicher einer Steuereinheit, einer Einheit zur Detektion von elektromagnetischen Störfeldern und, einer mit mindestens einer Antenne und/oder mindestens einer Telemetrieantenne Kommunikationseinheit, wobei der Informationsspeicher sowie die Einheit zur Detektion von elektromagnetischen Störfeldern mindestens mit der Kommunikationseinheit und der Steuereinheit direkt oder indirekt verbindbar sind und zumindest die Steuereinheit direkt mit der Kommunikationseinheit verbindbar ist, wobei
- die Steuereinheit ein erstes Signal von der Einheit zur Detektion von elektromagnetischen Störfeldern erhält, wenn die Einheit zur Detektion von elektromagnetischen Störfeldern ein von einem MRI-Gerät erzeugtes elektromagnetisches Störfeld detektiert und/oder die Steuereinheit ein zweites von einem MRI-Gerät über die Kommunikationseinheit gesendetes Signal erhält und wobei
- die Steuereinheit die Kommunikationseinheit veranlasst, ein drittes Signal über die mindestens eine Antenne und/oder die mindestens eine Telemetrieantenne an das MRI-Gerät auszusenden, wobei die Steuereinheit die Aussendung des dritten Signals entweder direkt nach Erhalt des ersten und/oder zweiten Signals oder zeitlich verzögert nach Erhalt des ersten und/oder zweiten Signals veranlasst, und wobei
- das dritte Signal ein zur Erzeugung einer Bildstörung am MRI-Gerät geeignetes Signal ist,
**dadurch gekennzeichnet, dass**
das IMD das Signal zur Bildstörung in der Auslesephase des MRI-Scanners auf einer entsprechenden Larmorfrequenz aussendet, wobei das Signal zur Bildstörung eine vorbestimmbare, modulierte Amplitude aufweist.

2. IMD nach Anspruch 1, **dadurch gekennzeichnet, dass** der Informationsspeicher Daten und/oder Parameter enthält, die eine gefahrlose MRI-Untersuchung oder eine MRI-Untersuchung mit verringerten Risiken erlauben, **dadurch gekennzeichnet, dass** diese Daten und/oder Parameter von der Kommunikationseinheit mit dem dritten Signal unter Verwendung der mindestens einen Antenne und/oder der mindestens einen Telemetrieantenne an das MRI-Gerät übertragen werden.

3. IMD nach einem der vorangegangenen Ansprüche, **dadurch gekennzeichnet, dass** das dritte Signal mindestens einen der folgenden Werte und/oder Parameter umfasst:
- maximal zulässige RF Leistung,
- maximal zulässige Gradientenleistung,
- maximal zulässige Scandauer, gegebenenfalls in Abhängigkeit von einem oder mehreren der anderen Parameter,
Patientenlage
- maximal zulässige Untersuchungszeit und/oder
- Parameter zur Synchronisation von MRI-Scans und/oder MRI-Auslesephasen und IMD Aktivität bezogen auf Signaldetektion und/oder Therapieabgabe.

4. IMD nach Anspruch 1, **dadurch gekennzeichnet, dass** das IMD die Larmorfrequenz aus einer Messung eines statischen Magnetfeldes und/oder der Frequenz der vorhergehenden elektromagnetischen Störung mittels der Einheit zur Detektion von elektromagnetischen Störfeldern bestimmt.

5. IMD nach Anspruch 4, **dadurch gekennzeichnet, dass** das IMD die Trägerfrequenz und/oder Modulationstyp und/oder Modulationsfrequenz und/oder Modulationstiefe des von der Kommunikationseinheit initiierten Signals zur Bildstörung aus den Messungen der Einheit zur Detektion von elektromagnetischen Störfeldern berechnet.

6. IMD nach einem der vorangegangenen Ansprüche, **dadurch gekennzeichnet, dass** das IMD keine interne Stromversorgung aufweist oder diese im Fälle einer Erkennung einer elektromagnetischen Störung nicht zur Verfügung steht und/oder zur Entlastung einer vorhandenen Batterie auf die Nutzung der Batterie zur Versorgung der Kommunikation mit einem MRI-Gerät verzichtet wird, wobei das dritte Signal mit der Kommunikationseinheit gesendet wird, indem durch das MRI-Gerät induzierte Energie verwendet wird, um den Energiebedarf der Kommunikationseinheit zum Senden des dritten Signals an das MRI-Gerät zu decken.

7. IMD nach einem der vorangegangenen Ansprüche, **dadurch gekennzeichnet, dass** die Antenne oder Antennen aus langgestreckten Subsystemen, wie, aber nicht beschränkt auf, Elektroden von Herzschrittmachern, Defibrillatoren/Cardiovertern, Geräten zur kardialen Resynchronisation oder Neurostimulatoren, und/oder einer bereits integrierten Antenne zur RF-Kommunikation bestehen.

8. IMD nach einem der vorangegangenen Ansprüche, **dadurch gekennzeichnet, dass** das IMD mindestens eine der folgenden Maßnahmen bei einer Erkennung von elektromagnetischen Störfeldern einleitet:
- das Wechseln in einen MRI-sicheren Zustand,
- ein verlängertes Verbleiben in einem MRI-sicheren oder gegenüber elektromagnetischen Störfeldern unempfindlichen Zustand, und
die Abgabe einer Therapie und/oder Detektion von elektrischen Zuständen des Gewebes nur in Zeitfenstern in denen keine elektromagnetischen Störfelder erkannt werden.

9. IMD nach einem der vorangegangenen Ansprüche, **dadurch gekennzeichnet, dass** die Einheit zur Detektion von elektromagnetischen Störfeldern mindestens einen der folgenden Sensoren oder Indikatoren umfasst:
- GMR-Sensor,
- MagFET-Sensor,
- Hallsensor,
- elektrooptische Wandler als Indikator,
- die Überwachung von Batteriespannungen während Kondensatorladeprozessen als Indikator,
- die Detektion von RF-Feldern als Indikator,
- die Detektion von magnetischen Grandientenfeldern als Indikator,
- die Detektion von durch elektromagnetische Felder induzierten Strömen als Indikator,
- die Detektion von spezifischen Vibrationen oder als Sensoren ausgelegte Bauteile zur Detektion von durch Lorentzkräfte induzierten Vibrationen als Indikator.

## Claims

1. An implantable medical device (IMD) comprising an information memory, a control unit, a unit for detecting electromagnetic interference fields, and a communication unit connectable to at least one antenna and/or at least one telemetry antenna, wherein the information memory and the unit for detecting electromagnetic interference fields are directly or indirectly connectable at least to the communication unit and the control unit, and at least the control unit is directly connectable to the communication unit, wherein
- the control unit receives a first signal from the unit for detecting electromagnetic interference fields when the unit for detecting electromagnetic interference fields detects an electromagnetic interference field generated by an MRI device, and/or the control unit receives a second signal transmitted from an MRI device via the communication unit, and wherein
- the control unit causes the communication unit to transmit a third signal to the MRI via the at least one antenna and/or the at least one telemetry antenna, wherein the control unit causes the transmission of the third signal either directly after receiving the first and/or the second signal, or with a time delay after receiving the first and/or second signal, and wherein
- the third signal is a signal suitable for generating an image interference on the MRI device,
**characterized in that**
in the read-out phase, the IMD transmits the signal for image interference on a corresponding Larmor frequency, wherein the signal for image interference has a predeterminable modulated amplitude.

2. The IMD according to claim 1, **characterized in that** the information memory contains data and/or parameters that allow for a risk-free MRI examination or an MRI examination with reduced risks, **characterized in that** said data and/or parameters are transmitted to the MRI device from the communication unit with the third signal by using the at least one antenna and/or the at least one telemetry antenna.

3. The IMD according to any one of the preceding claims, **characterized in that** the third signal comprises at least one of the following values and/or parameters:
- maximum allowable RF power,
- maximum allowable gradient performance,
- maximum allowable scan duration, if applicable depending on one or more of the other parameters,
- position of the patient,
- maximum allowable examination time and/or
- parameters for synchronizing MRI scans and/or MRI read-out phases and IMD activity with regard to signal detection and/or delivery of therapy.

4. The IMD according to claim 1, **characterized in that** the IMD determines the Larmor frequency through a measurement of a static magnetic field and/or of the frequency of the previous electromagnetic interference by means of the unit for detecting electromagnetic interference fields.

5. The IMD according to claim 4, **characterized in that** the IMD calculates the carrier frequency and/or modulation type and/or modulation frequency and/or modulation depth of the image interference signal initiated by the communication unit from the measurements of the unit for detecting electromagnetic interference fields.

6. The IMD according to any one of the preceding claims, **characterized in that** the IMD has no internal power supply or said power supply is not available in the case of detection of an electromagnetic interference, and/or in order to remove load from an available battery, use of the battery for powering the communication with an MRI device is dispensed with, wherein the third signal is transmitted with the communication unit by using power induced by the MRI device so as to cover the power requirement of the communication unit for transmitting the third signal to the MRI device.

7. The IMD according to any one of the preceding claims, **characterized in that** the antenna or the antennas consists of an elongated subsystem such as, but not limited to, electrodes of cardiac pacemakers, defibrillators/cardioverters, devices for cardiac resynchronization or neurostimulators, and/or an already integrated antenna for RF communication.

8. The IMD according to anyone of the preceding claims, **characterized in that** upon detection of electromagnetic interference fields, the IMD initiates at least one of the following measures:
- switching to an MRI-safe state,
- remaining for an extended time in an MRI-safe state or a state that is insensitive to electromagnetic interference fields, and
- delivering a therapy and/or detecting electrical states of the tissue only within time windows in which no electromagnetic interference fields are detected.

9. The IMD according to any one of the preceding claims, **characterized in that** the unit for detecting electromagnetic interference fields comprises at least one of the following sensors or indicators:
- GMR sensor,
- MagFET sensor,
- Hall sensor,
- electro-optical converter, as an indicator,
- monitoring battery voltages during capacitor charging processes, as an indicator,
- detecting FR fields, as an indicator,
- detecting magnetic gradient fields, as an indicator,
- detecting currents induced by electromagnetic fields, as an indicator,
- detecting specific vibrations or components designed for detecting vibrations induced by Lorentz forces, as an indicator.

## Revendications

1. Dispositif médical implantable (IMD) avec une mémoire d'information, une unité de commande, une unité pour la détection de champs électromagnétiques parasites et une unité de communication apte à être reliée à au moins une antenne et/ou au moins une antenne de télémétrie, dans lequel la mémoire d'information ainsi que l'unité pour la détection de champs électromagnétiques parasites peuvent être reliées directement ou indirectement à au moins l'unité de communication et l'unité de commande, et au moins l'unité de commande pouvant être reliée directement à l'unité de communication, dans lequel
- l'unité de commande reçoit un premier signal venant de l'unité pour la détection de champs électromagnétiques parasites lorsque l'unité pour la détection de champs électromagnétiques parasites détecte un champ électromagnétique parasite produit par un dispositif d'IRM, et/ou l'unité de commande reçoit un deuxième signal envoyé par un dispositif d'IRM par le biais de l'unité de communication, et dans lequel
- l'unité de commande ordonne à l'unité de communication d'envoyer un troisième signal au dispositif d'IRM à travers la ou les antennes et/ou la ou les antennes de télémétrie, dans lequel l'unité de commande ordonne la transmission du troisième signal soit directement après l'obtention du premier et/ou du deuxième signal, soit plus tard après l'obtention du premier et/ou du deuxième signal, et dans lequel
- le troisième signal est un signal adapté pour produire une perturbation d'image sur le dispositif d'IRM,
**caractérisé en ce que**
pendant la phase de lecture, le dispositif médical implantable (IMD) envoie le signal pour la perturbation d'image à une fréquence de Larmor correspondante, dans lequel le signal pour la perturbation d'image présente une amplitude modulée prédéfinissable.

2. Dispositif médical implantable (IMD) selon la revendication 1, **caractérisé en ce que** la mémoire d'information contient des données et/ou des paramètres permettant d'effectuer un examen par IRM sans risque ou un examen par IRM avec des risques réduits, **caractérisé en ce que** lesdites données et/ou paramètres sont transmis au dispositif d'IRM par l'unité de communication avec le troisième signal, à l'aide de la ou des antennes et/ou de la ou des antennes de télémétrie.

3. Dispositif médical implantable (IMD) selon l'une des revendications précédentes, **caractérisé en ce que** le troisième signal comprend au moins l'une des valeurs et/ou l'un des paramètres suivants :
- puissance RF maximale autorisée,
- puissance de gradient maximale autorisée,
- durée de balayage maximale autorisée, éventuellement en fonction de l'un ou de plusieurs des autres paramètres,
- position du patient,
- durée d'examen maximale autorisée et/ou
- paramètres pour la synchronisation de balayages d'IRM et/ou de phases de lecture d'IRM et de l'activité du dispositif médical implantable (IMD) par rapport à la détection de signaux et/ou de la délivrance du traitement.

4. Dispositif médical implantable (IMD) selon la revendication 1, **caractérisé en ce que** le dispositif médical implantable (IMD) détermine la fréquence de Larmor à partir d'une mesure d'un champ magnétique statique et/ou de la fréquence de la perturbation électromagnétique précédente, au moyen de l'unité pour la détection de champs électromagnétiques parasites.

5. Dispositif médical implantable (IMD) selon la revendication 4, **caractérisé en ce que** le dispositif médical implantable (IMD) calcule la fréquence porteuse et/ou le type de modulation et/ou la fréquence de modulation et/ou la profondeur de modulation du signal de perturbation de l'image initié par l'unité de communication à partir de la mesure de l'unité pour la détection de champs électromagnétiques parasites.

6. Dispositif médical implantable (IMD) selon l'une des revendications précédentes, **caractérisé en ce que** le dispositif médical implantable (IMD) ne comporte pas d'alimentation électrique interne ou que celle-ci n'est pas disponible dans le cas d'une détection de perturbation électromagnétique et/ou que l'utilisation de la batterie pour l'alimentation de la communication avec un dispositif d'IRM est interrompue pour soulager une batterie existante, dans lequel le troisième signal est envoyé avec l'unité de communication en utilisant l'énergie induite par le dispositif d'IRM, afin de couvrir le besoin énergétique de l'unité de communication pour l'envoi du troisième signal au dispositif d'IRM.

7. Dispositif médical implantable (IMD) selon l'une des revendications précédentes, **caractérisé en ce que** l'antenne ou les antennes sont constituées d'un sous-système allongé, tel que des électrodes de stimulateur cardiaque, des défibrillateurs/cardioverteurs, des dispositifs pour la resynchronisation cardiaque ou des neuro-stimulateurs, mais sans se limiter à ceux-ci, et/ou d'une antenne déjà pré-intégrée pour la communication RF.

8. Dispositif médical implantable (IMD) selon l'une des revendications précédentes, **caractérisé en ce que** le dispositif médical implantable (IMD) prend au moins l'une des mesures suivantes lors de la détection de champs électromagnétiques parasites :
- le passage à l'état d'IRM sûr,
- un temps prolongé dans un état d'IRM sûr ou dans un état insensible aux champs électromagnétiques parasites, et
- la délivrance d'un traitement et/ou la détection d'états électriques du treillis seulement dans des périodes durant lesquelles aucun champ électromagnétique parasite n'est détecté.

9. Dispositif médical implantable (IMD) selon l'une des revendications précédentes, **caractérisé en ce que** l'unité pour la détection de champs électromagnétiques parasites comprend au moins l'un des capteurs ou indicateurs suivants :
- capteur GMR,
- capteur MagFET,
- capteur Hall,
- convertisseur électro-optique comme indicateur,
- surveillance des tensions de batterie pendant les opérations de charge du condensateur comme indicateur,
- détection de champs FR comme indicateur,
- détection de champs magnétiques à gradients comme indicateur,
- détection de courants induits par des champs électromagnétiques comme indicateur,
- détection de vibrations spécifiques ou de composants conçus comme des capteurs pour la détection de vibrations induites par des forces de Lorentz, comme indicateur.
